# NEUE EUROPÄISCHE PATENTSCHRIFT

(11) **EP 1 758 555 B2**
(45) Veröffentlichungstag und Bekanntmachung des Hinweises auf die Entscheidung über den Einspruch: **07.09.2011**
(45) Hinweis auf die Patenterteilung: 25.02.2009
(21) Anmeldenummer: 05756084.9
(22) Anmeldetag: 21.06.2005
(51) Int. Cl.: A61K 9/06, A61K 47/06

(54) **TRITERPENHALTIGER OLEOGELBILDNER, TRITERPENHALTIGES OLEOGEL UND VERFAHREN ZUR HERSTELLUNG EINES TRITERPENHALTIGEN OLEOGELS**
TRITERPENE-CONTAINING OLEOGEL-FORMING AGENT, TRITERPENE-CONTAINING OLEOGEL AND METHOD FOR PRODUCING A TRITERPENE-CONTAINING OLEOGEL
AGENT OLEOGELIFIANT CONTENANT DU TRITERPENE, OLEOGEL CONTENANT DU TRITERPENE ET PROCEDE DE FABRICATION D'UN AGENT OLEOGELIFIANT CONTENANT DU TRITERPENE

(30) Priorität: 22.06.2004 DE 102004030044
(43) Veröffentlichungstag der Anmeldung: 07.03.2007
(73) Patentinhaber: BIRKEN GMBH, 75223 Niefern-Öschelbronn (DE)
(72) Erfinder: SCHEFFLER, Armin, 75229 Niefern-Öschelbronn (DE)
(74) Vertreter: Bickel, Michael
(86) Internationale Anmeldenummer: PCT/EP2005/006710
(87) Internationale Veröffentlichungsnummer: WO 2005/123037

(56) Entgegenhaltungen:
- EP-A- 1 410 790
- WO-A-00/62751
- WO-A-03/043594
- US-A1- 2003 087 789
- SHRIKHANDE B K ET AL: "DEVELOPMENT AND EVALUATION OF ANTI-INFLAMMATORY OLEOGELS OF BOSEWELLIA SERRATA (GUGUL) AND CURCUMA LONGA (TURMERIC)" INDIAN DRUGS, Bd. 38, Nr. 12, Dezember 2001 (2001-12), Seiten 613-616, XP008005371

## Beschreibung

Die vorliegende Erfindung betrifft einen Oleogelbildner, ein Oleogel mit diesem Gelbildner und ein Verfahren zur Herstellung eines Oleogels.

Shrikhande et al.: "Development and Evaluation of Anti-Inflammatory Oleogels of Bosewellia Serrate (Gugul) and Curcuma Longa (Turmeric)", Indian Drugs, Bd. 38, Nr. 12, Dezember 2001, Seiten 613 bis 616, beschreibt Oleogele, die Pf lanzenextrakte Boswellia serrata (Weihrauch) und Curcuma longa (Curcuma) aufweisen. Als Gelbildner dienen bei diesen Oleogelen kolloidale Kieselsäuren.

Gele sind feindisperse Systeme aus einer flüssigen und einer festen Phase, wobei die feste Phase ein zusammenhängendes dreidimensionales Gerüst bildet und die beiden Phasen sich vollständig durchdringen. Man unterscheidet im wesentlichen zwischen hydrophilen Gelen und hydrophoben Gelen. Letztere werden auch als Oleogele bezeichnet. Oleogele basieren auf einer unpolaren Flüssigkeit, beispielsweise einem Öl, einem Wachs oder einem Paraffin, der ein Gelbildner zur Erzielung der gewünschten physikalischen Eigenschaften beigefügt ist.

Derartige Oleogele können je nach Zusammensetzung unterschiedlichsten Zwecken dienen.

Besonders im pharmazeutischen Bereich finden Oleogele Verwendung für topische Anwendungen. Bei diesen pharmazeutischen Oleogelen ist ein Gelbildner neben den pharmazeutisch wirksamen Substanzen in dem Gel vorhanden. Ein häufig verwendeter Gelbildner für pharmazeutische Oleogele ist hochdisperses Siliziumdioxid, das unter dem Handelsnamen Aerosil^{®} erhältlich ist. Oleogele besitzen eine ausgeprägte Thixotropie, d.h. sie verflüssigen sich bei mechanischer Einwirkung und verfestigen sich anschließend wieder. Andere Gele, beispielsweise Gele mit Pektin als Gelbildner, vernetzen unter Säureeinwirkung, wieder andere gelieren temperaturabhängig, wie beispielsweise Gelatine.

Auch im technischen Bereich finden Oleogele Verwendung. Ein Beispiel hierfür sind unpolare Auftragsmittel (tropffreie Farben). Als Gelbildner kann auch für diese Anwendungen hochdisperses Siliziumdioxid zum Einsatz kommen. Dieser mineralische Gelbildner besitzt für technische Anwendungen den Nachteil, dass er bei einer thermischen Verwertung eines mit einem solchen Oleogel behandelten Produkts nicht aschefrei verbrennt.

Aufgabe der vorliegenden Erfindung ist es, einen Oleogelbildner, der selbst pharmazeutisch aktiv ist und der aschefrei verbrennt, ein Oleogel mit einem solchen Gelbildner und ein Verfahren zur Herstellung eines Oleogels mit einem solchen Gelbildner zur Verfügung zu stellen.

Diese Aufgabe wird durch einen Oleogelbildner mit den Merkmalen des Anspruchs 1, ein Oleogel mit den Merkmalen des Anspruchs 9 und ein Verfahren mit den Merkmalen des Anspruchs 22 gelöst.

Der Oleogelbildner weist erfindungsgemäß wenigstens ein hochdisperses Triterpen auf.

Triterpene, wie Betulin, Lupeol, Betulinsäure, Oleanolsäure und ähnliche Verbindungen, sind nachwachsende Rohstoffe, die in z.B. Birkenrinde vorkommen. Betulin, Betulinsäure, Lupeol und Oleanolsäure sind dabei pentazyklische Triterpene, die ersten drei mit einem Lupan-Gerüst, letzteres mit einem Oleanangerüst. Das charakteristische Merkmal der Lupan-Gruppe ist ein Ring mit fünf Kohlenstoffatomen innerhalb des pentazyklischen Systems, der eine α-Isopentenylgruppe an der Position C-19 besitzt.

Ein Verfahren zur Gewinnung von Triterpenen aus Pflanzenbestandteilen, insbesondere von Betulin aus Birkenrinde, ist beispielsweise in der WO 2001/72315 A1 oder der WO 2004/016336 A1 beschrieben.

Die pharmakologischen Eigenschaften von Triterpenen, insbesondere von Betulin, machen den erfindungsgemäßen triterpenhaltigen Oleogelbildner für die Herstellung kosmetischer und pharmazeutischer Oleogele besonders interessant.

Die antiseptischen Eigenschaften von Betulin wurden bereits 1899 nachgewiesen, es wurde daher für die Sterilisierung von Wundverbänden und Pflastern verwendet (Wheeler, J., (1899), Pharm. J., Die Darstellung des Betulin durch Sublimation, 494, Ref. Chem. Centr. 1900 I, S. 353).

Ferner konnte für Betulin und Betulinderivate eine entzündungshemmende, kortisonähnliche Wirkung ebenso wie eine zytostatische Wirkung bei Verwendung verschiedener Tumorzellinien in vitro nachgewiesen werden (Carmen Recio, M., et. al. (1995), Investigations on the steroidal anti-inflammatory activity of triterpenoids from Diospyros leucomelas, Planta Med. 61, S. 9-12; Yasukawa, K., et. al., (1991), Sterol and triterpene derivates from plants, Oncogene 48, S. 72-76).

Eine antivirale Wirkung von Betulin bei Herpes-Simplex-Viren ist in der US 5,750,578 beschrieben. Die US 2002/0119935 A1 beschreibt die Wirkung von Triterpenen bei bakteriellen Infektionen und die US 2002/0128210 A1 beschreibt die Wirkung von Triterpenen bei Pilzinfektionen.

Die mittlere Partikelgröße des wenigstens einen Triterpens in dem Oleogelbildner beträgt weniger als 50µm. Besonders bevorzugt ist die mittlere Partikelgröße kleiner als 10µm oder gar kleiner als 100nm, um hervorragende Gelbildungseigenschaften zu erzielen. Man spricht in diesem Zusammenhang von einer Feindispersität, wenn die Partikelgröße zwischen 100nm und 10µm beträgt, und von einer Kolloiddispersität, wenn die Partikelgröße zwischen 1nm und 100nm beträgt.

Der Anteil der Sekundäragglomerate des wenigstens einen Triterpens in dem Oleogelbildner beträgt vorzugsweise weniger als 20 Gew.%. Idealerweise liegt eine homogene Partikelgrößenverteilung vor, also eine Normalverteilung der Häufigkeit einzelner Partikelgrößen. Denn möglicherweise wirkt sich das Vorhandensein von Sekundäragglomeraten negativ auf die Gelbildungseigenschaften eines Pulvers aus, wie der Artikel Knop, Reimann: "Kolloidale Kieselsäuren als Gelbildner", GO-VI-Verlag, 2001, vermuten lässt.

Auswirkungen auf die Eigenschaften des wenigstens einen hochdispersen Triterpens als Oleogelbildner zu wirken, kann auch die spezifische Oberfläche dieses Triterpens haben, wobei Versuche gezeigt haben, dass sich die Gelbildungseigenschaften mit zunehmender spezifischer Oberfläche verbessern. Diese spezifische Oberfläche des wenigstens einen Triterpens beträgt bei einer Ausgestaltung zwischen 1 m²/g und 500 m²/g und liegt vorzugsweise zwischen 10 m²/g und 100 m²/g und besonders bevorzugt zwischen 20 m²/g und 50 m²/g.

Der in Form eines mikronisierten triterpenhaltigen Pulvers vorliegende Oleogelbildner kann neben Triterpenen, wie beispielsweise Betulin, Betulinsäure, Lupeol oder Allobetulin, auch einen Anteil anderer Stoffe umfassen, beispielsweise solche Stoffe, die in triterpenhaltigen Pflanzenbestandteilen, wie beispielsweise Birkenrinde, aus denen Triterpene extrahiert werden können, ebenfalls in einem gewissen Anteil natürlich vorhanden sind. Der Triterpenanteil in dem erfindungsgemäßen Oleogelbildner beträgt vorzugsweise mehr als 80 Gew.%, besonders bevorzugt mehr als 90 Gew.% bezogen auf das Gewicht des Oleogelbildners. Vorteilhafterweise beträgt der Betulinanteil bezogen auf den Triterpenanteil dabei mehr als 80 Gew.%.

Der erfindungsgemäße triterpenhaltige Oleogelbildner ist auch für technische Anwendungen einsetzbar, beispielsweise in unpolaren Auftragsmitteln. Er besitzt für derartige Anwendungen den Vorteil, dass er - anders als mineralische Gelbildner - bei einer thermischen Verwertung aschefrei verbrennt.

Das erfindungsgemäße Oleogel enthält:
- eine unpolare Flüssigkeit mit einem Anteil zwischen 80 Gew.% und 99 Gew.% bezogen auf das Gesamtgewicht des Gels, und
- als Gelbildner den zuvor erläuterten triterpenhaltigen Oleogelbildner mit einem Anteil zwischen 1 Gew.% und 20 Gew.%, vorzugsweise zwischen 3 Gew.% und 15 Gew.%, besonders bevorzugt zwischen 6 Gew.% und 12 Gew.%, bezogen auf das Gesamtgewicht des Gels.

Der Vorteil dieser halbfesten Zubereitung in Form eines Oleogels liegt in der Einfachheit seiner Rezeptur, wobei das Triterpen gleichzeitig als pharmazeutisch wirksame Substanz und als Gelbildner funktioniert, so dass auf zusätzliche Gelbildner verzichtet werden kann. Das Oleogel ist dadurch besonders für allergiegefährdete Haut geeignet.

Unter Verwendung des erläuterten triterpenhaltigen hochdispersen, bevorzugt feindispersen oder kolloiddispersen, Pulvers als Oleogelbildner mit einem Triterpengehalt in dem erläuterten Konzentrationsbereich und mit der angegebenen mittleren Partikelgröße lässt sich also ein Gel herstellen, das neben dem pharmazeutisch wirksamen, in Pulverform vorliegenden wenigstens einen Triterpen und der unpolaren Flüssigkeit keine weiteren Bestandteile enthalten muss. Triterpene besitzen in unpolaren Flüssigkeiten eine Löslichkeit von weniger als 0,5%, so dass die Triterpene in dem Gel überwiegend als ungelöste Feststoffpartikel vorliegen.

Selbstverständlich besteht jedoch auch die Möglichkeit, dem Oleogel neben dem im Gelbildner vorhandenen Triterpen weitere pharmazeutisch aktive Substanzen beizufügen.

Die Vorteile eines Oleogels mit einem triterpenhaltigen Oleogelbildner sind je nach Anwendungsgebiet vielfältig.

Für den kosmetisch-pharmazeutischen Bereich wird damit eine neue, halbfeste Zubereitung zur Verfügung gestellt, die gegenüber wasserhaltigen Zubereitungen besonders gut bei trockener Haut und auf den Lippen anwendbar ist. Die topische Anwendung des erfindungsgemäßen Oleogels ist besonders vorteilhaft bei allergiegefährdeten Menschen, weil keine anderen Gelbildner erforderlich sind. Andererseits lässt sich das Oleogel ohne Zusätze auch als pharmazeutische Grundlage verwenden, in welche andere lipophile und mit Wasser auch hydrophile Wirk- oder Hilfsstoffe besonders leicht eingemischt werden können.

Für den technischen Bereich wird eine thixotrope Zusammensetzung mit einem nicht mineralischen, und damit aschefrei verbrennbaren Oleogelbildner zur Verfügung gestellt. Ein Anwendungsbereich sind beispielsweise unpolare Auftragsmittel (tropffreie Farben), mit einer dank des erfindungsgemäßen Oleogelbildners erhöhten Thixotropie. Der Gelbildner fügt zugleich die für Triterpene bekannten antiseptische Eigenschaften und den für Triterpene bekannten Lichtschutz hinzu.

Der Anteil der unpolaren Flüssigkeit in dem Oleogel beträgt vorzugsweise zwischen 88 Gew.% und 94 Gew.%, und der Anteil des triterpenhaltigen Pulvers beträgt vorzugsweise zwischen 6 Gew.% und 12 Gew.%.

Als unpolare Flüssigkeit für das Oleogel eignen sich beliebige unpolare Flüssigkeiten, wie beispielsweise pflanzliche, tierische oder synthetische Öle, Wachse und Paraffine. Die unpolare Flüssigkeit ist beispielsweise ein pflanzliches Öl, wie beispielsweise Sonnenblumenöl, Olivenöl, Avocadoöl, Mandelöl, oder eine Mischung dieser Öle.

Das erfindungsgemäße Oleogel besitzt eine nur wenig temperaturabhängige Viskosität, jedoch ein ausgeprägtes Thixotropieverhalten, wodurch das Gel einfach zu lagern und anzuwenden ist.

Der Oleogelbildner in Form des hochdispersen, bevorzugt feindispersen oder kolloiddispersen, Triterpenpulvers kann auch als Verdickungsmittel dienen, wenn er in der zu verdickenden Flüssigkeit in Konzentrationen unterhalb der Geliergrenze eingesetzt wird, also unterhalb der Konzentration, die erforderlich wäre, um aus der Flüssigkeit und dem Triterpen ein oleogel zu bilden.

So besteht die Möglichkeit, das hochdisperse Triterpen der unpolaren Flüssigkeit in einer Konzentration zuzugeben, die unterhalb der Geliergrenze liegt, die also unterhalb der für eine Gelbildung erforderlichen Konzentration liegt. Ergebnis ist ein Oleosol, also eine zähflüssige Zubereitung in der das hochdisperse, bevorzugt feindisperse oder kolloiddisperse, Triterpen als Verdickungsmittel wirkt.

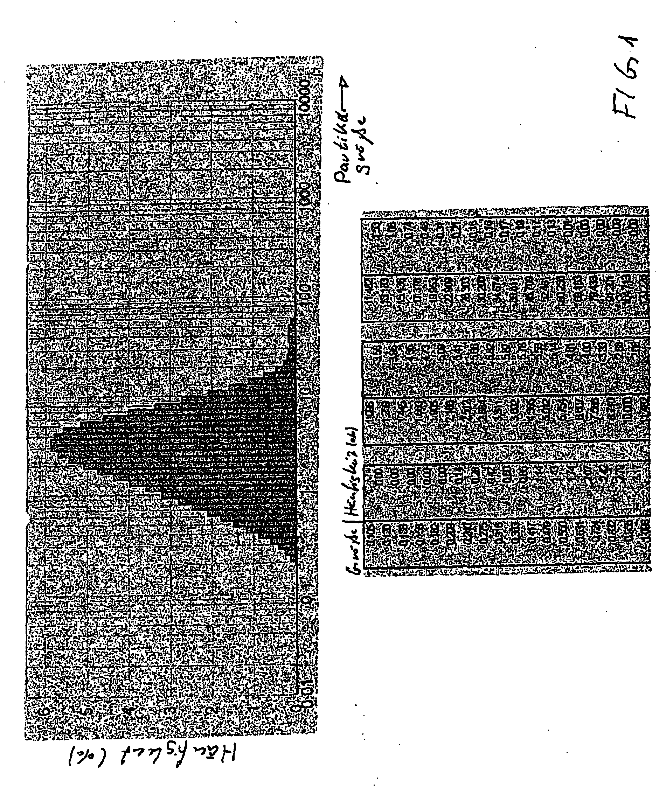

Die vorliegende Erfindung wird nachfolgend anhand eines Ausführungsbeispiels unter Bezugnahme auf die beigefügte Figur erläutert.

Die Figur zeigt im oberen Bereich in Form eines Histogramms die homogene Partikelgrößenverteilung einer Probe eines erfindungsgemäßen hochdispersen Oleogelbildners. Die der Kurve zugrundeliegenden Messwerte sind in Form einer Tabelle im unteren Bereich der Figur dargestellt. Ein Wert für die Häufigkeitsverteilung im rechten Teil der Tabelle ist dabei jeweils auf ein Intervall von zwei Partikelgrößen bezogen, die im linken Teil der Tabelle nach oben und nach unten versetzt zu dem zugehörigen Häufigkeitswert dargestellt sind. So ist der Tabelle beispielsweise zu entnehmen, dass der Anteil der Partikel mit einer Größe zwischen 0,209µm und 0,240µm in der untersuchten Probe 0,14% beträgt.

Die Partikelgrößen der Probe liegen zwischen 0,2µm und 60,2µm, das Maximum der Größenverteilung liegt zwischen 2,5µm und 5µm.

Die Verteilung der Partikelgrößen für die untersuchte Probe ist nahezu homogen, d.h. die Häufigkeit der Verteilung nimmt für Durchmesser, die kleiner sind als das Maximum, das etwa bei 3,5 µm liegt, stetig zu und nimmt für Durchmesser, die größer sind als das Maximum, stetig ab. Lediglich für Partikel mit einer Größe zwischen etwa 34 µm und 45 µm nimmt die Häufigkeit wieder etwas zu. Diese Zunahme dürfte auf Sekundäragglomerate zurückzuführen sein, also Ansammlungen von Partikeln, die sich erst nach der eigentlichen Kristallisation gebildet haben oder die durch das Zusammenwachsen zweier oder mehrerer zunächst unabhängig voneinander kristallisierter Kristalle entstanden sind.

Gemäß gaschromatographischer Analyse enthält dieses Pulver 85 Gew.% Betulin, 5 Gew.% Betulinsäure, 3% Oleanolsäure, 0,7 Gew.% Lupeol und 6,3 Gew.% übrige Triterpenderivate.

Unter Verwendung dieses hochdispersen Pulvers als Gelbildner wurde ein Oleogel hergestellt, indem das Pulver mit 9 Gew.% bezogen auf das Gesamtgewicht des Oleogels mit Sonnenblumenöl vermischt wurde. Ergebnis war ein stabiles halbfestes Gel mit stark ausgeprägter Thixotropie.

Dieses so erzeugte Oleogel eignet sich zur Behandlung verschiedenster Hautkrankheiten bei Mensch und Tier. Beispiele hierfür sind aktinische Keratosen und Basaliome beim Mensch und Euterentzündung bei Säugetieren.

Das als Oleogelbildner wirkende, wenigstens ein Triterpen enthaltende Pulver kann mittels beliebiger herkömmlicher Extraktionsverfahren aus Pflanzenbestandteilen gewonnen werden. Sofern das durch solche Extraktionsverfahren gewonnene Pulver nicht mit der für die Gelbildungseigenschaften erforderlichen Dispergierbarkeit, mittleren Partikelgröße und homogenen Partikelgrößenverteilung vorliegt, kann das Pulver verschiedenen Verfahren unterzogen werden, um zu der gewünschten Partikelgröße, Homogenität und Dispergierbarkeit zu gelangen. Hierfür sind dem auf diesem Gebiet geschulten Fachmann verschiedene Verfahren bekannt, von denen einige nachfolgend kurz erläutert werden.

Wenn die Partikelgröße in dem Pulver zu hoch ist, eignen sich Prall- oder Gravitationsverfahren zur Verkleinerung der Partikel.

Darüber hinaus besteht die Möglichkeit, das Pulver in einem geeignetem Lösungsmittel, beispielsweise Tetrahydrofuran (THF), zu lösen und anschließend erneut zu kristallisieren. Diese Kristallisation kann beispielsweise durch Sprühtrocknung oder Abkühlen eines gesättigten Lösungsmittels erfolgen. Die Partikelgröße kann dabei über die Kristallisationsbedingungen eingestellt werden. Die Kristallisationsbedingungen sind bei einer Sprühtrocknung beispielsweise vom Durchmesser einer Düse, über welche das Triterpen-Lösungsmittel-Gemisch versprüht wird, und von der Temperatur und dem Druck in einer Kammer, in die das Gemisch gesprüht wird, abhängig. Beim Kristallisieren durch Abkühlen einer gesättigten Lösung sind die Kristallisationsbedingungen vom zeitlichen Temperaturgradienten während des Abkühlens und von der Triterpenkonzentration in der Lösung abhängig.

Es hat sich gezeigt, dass besonders kleine Triterpenpartikel mit großer spezifischer Oberfläche dadurch erhalten werden können, dass zu einem gesättigten Triterpen-Lösungsmittel-Gemisch kaltes Lösungsmittel beigemischt wird. Diese Beimischung des kalten Lösungsmittels führt dazu dass die Lösung abkühlt, wodurch die Triterpene auskristallisieren. Gleichzeitig reduziert das zugeführte kalte Lösungsmittel die Triterpenkonzentration in dem Lösungsmittel mit dem Ergebnis, dass eher kleine Kristalle entstehen, was im Hinblick auf die Gelbildungseigenschaften vorteilhaft ist.

Schließlich besteht auch die Möglichkeit ein vorhandenes Pulver zu klassieren, um ein Pulver mit einer gewünschten Größenverteilung zu erhalten.

## Patentansprüche

1. Verwendung wenigstens eines hoch dispersen Triterpens mit einer mittleren Partikelgröße von weniger als 50µm als Oleogelbildner in einem Oleogel.

2. Verwendung nach Anspruch 1, bei dem die mittlere Partikelgröße des wenigstens einen Triterpens weniger als 10µm beträgt.

3. Verwendung nach einem der vorangehenden Ansprüche, bei dem ein Anteil an Sekundäragglomeraten des wenigstens einen Triterpens weniger als 20 Gew.% beträgt.

4. Verwendung nach Anspruch 3, bei dem das wenigstens eine Triterpen eine homogene partikelgrößenverteilung besitzt.

5. Verwendung nach einem der vorangehenden Ansprüche, bei dem die spezifische Oberfläche des wenigstens einen Triterpens zwischen 1 m²/g und 500 m²/g beträgt.

6. Verwendung nach Anspruch 5, bei dem die spezifische Oberfläche des wenigstens einen Triterpens zwischen 10 m²/g und 100 m²/g beträgt.

7. Verwendung nach Anspruch 6, bei dem die spezifische Oberfläche des wenigstens einen triterpens zwischen 20 m²/g und 50 m²/g beträgt.

8. Verwendung nach einem der vorangehenden Ansprüche, bei dem das wenigstens eine Triterpen einen Anteil von mehr als 80 Gew.% Betulin umfasst.

9. Oleogel, das folgende Bestandteile enthält:
- eine unpolare Flüssigkeit mit einem Anteil zwischen 80 Gew.% und 99 Gew.% bezogen auf das Gesamtgewicht des Gels,
- wenigstens ein hoch disperses Triterpen als Oleogelbildner, das eine mittlere Partikelgröße von weniger als 50 µm aufweist, mit einem Anteil zwischen 1 Gew.% und 20 Gew.% bezogen auf das Gesamtgewicht des Gels.

10. Oleogel nach Anspruch 9, bei dem die mittlere Partikelgröße des wenigstens einen Triterpens weniger als 10µm beträgt.

11. Oleogel nach einem der Ansprüche 9 oder 10, bei dem ein Anteil an Sekundäragglomeraten des wenigstens einen Triterpens weniger als 20 Gew.% beträgt.

12. Oleogel nach Anspruch 11, bei dem das wenigstens eine Triterpen eine homogene Partikelgrößenverteilung besitzt.

13. Oleogel nach einem der Ansprüche 9 bis 12, bei dem die spezifische Oberfläche des wenigstens einen Triterpens zwischen 1 m²/g und 500 m²/g beträgt.

14. Oleogel nach Anspruch 13, bei dem die spezifische Oberfläche des wenigstens einen Triterpens zwischen 10 m²/g und 100 m²/g beträgt.

15. Oleogel nach Anspruch 14, bei dem die spezifische Oberfläche des wenigstens einen Triterpens zwischen 20 m²/g und 50 m²/g beträgt.

16. Oleogel nach einem der Ansprüche 9 bis 15, bei dem das wenigstens eine Triterpen einen Anteil von mehr als 80 Gew.% Betulin umfasst.

17. Oleogel nach einem der Ansprüche 9 bis 16, bei dem der Anteil des Oleogelbildners zwischen 3 Gew.% und 15 Gew.% beträgt.

18. Oleogel nach Anspruch 17, bei dem der Anteil der unpolaren Flüssigkeit zwischen 88 Gew.% und 94 Gew.% und der Anteil des Oleogelbildners zwischen 6 Gew.% und 12 Gew.% beträgt.

19. Oleogel nach einem der Ansprüche 9 bis 18, bei dem die unpolare Flüssigkeit ein pflanzliches, tierisches, mineralisches oder synthetisches Öl ist.

20. Oleogel nach Anspruch 19, bei dem das Öl eines der folgenden oder eine Mischung der folgenden Pflanzenöle ist: Sonnenblumenöl, Olivenöl, Avocadoöl, Mandelöl.

21. Oleogel nach einem der Ansprüche 9 bis 18, bei dem die unpolare Flüssigkeit ein Wachs oder ein Paraffin ist.

22. Verfahren zur Herstellung eines Oleogels, das das Vermischen nur folgender Bestandteile umfasst:
- eine unpolare Flüssigkeit mit einem Anteil zwischen 80 Gew.% und 99 Gew.% bezogen auf das Gesamtgewicht des Gels,
- wenigstens ein hoch disperses Triterpen als Oleogelbildner, das eine mittlere Partikelgröße von weniger als 50 µm aufweist, mit einem Anteil zwischen 1 Gew.% und 20 Gew.% bezogen auf das Gesamtgewicht des Gels.

23. Verfahren nach Anspruch 22, bei dem die mittlere Partikelgröße des wenigstens einen Triterpens weniger als 10µm beträgt.

24. Verfahren nach einem der Ansprüche 22 oder 23, bei dem ein Anteil an Sekundäragglomeraten des wenigstens einen Triterpens weniger als 20 Gew.% beträgt.

25. Verfahren nach Anspruch 24, bei dem das wenigstens eine Triterpen eine homogene Partikelgrößenverteilung besitzt.

26. Verfahren nach einem der Ansprüche 22 bis 25, bei dem die spezifische Oberfläche des wenigstens einen Triterpens zwischen 1 m²/g und 500 m²/g beträgt.

27. Verfahren nach Anspruch 26, bei dem die spezifische Oberfläche des wenigstens einen Triterpens zwischen 10 m²/g und 100 m²/g beträgt.

28. Verfahren nach Anspruch 27, bei dem die spezifische Oberfläche des wenigstens einen Triterpens zwischen 20 m²/g und 50 m²/g beträgt.

29. Verfahren nach einem der Ansprüche 22 bis 28, bei dem das wenigstens eine Triterpen einen Anteil von mehr als 80 Gew.% Betulin umfasst.

30. Verfahren nach einem der Ansprüche 22 bis 29, bei dem der Anteil des Oleogelbildners zwischen 3 Gew.% und 15 Gew.% beträgt.

31. Verfahren nach Anspruch 30, bei dem der Anteil der unpolaren Flüssigkeit zwischen 88 Gew.% und 94 Gew.% und der Anteil des Oleogelbildners zwischen 6 Gew.% und 12 Gew.% beträgt.

32. Verfahren nach einem der Ansprüche 22 bis 31, bei dem die unpolare Flüssigkeit ein pflanzliches, tierisches oder synthetisches Öl ist.

33. Verfahren nach Anspruch 32, bei dem das Öl eines der folgenden oder eine Mischung der folgenden Pflanzenöle ist: Sonnenblumenöl, Olivenöl, Avocadoöl, Mandelöl.

34. Verfahren nach einem der Ansprüche 22 bis 29 und 31, bei dem die unpolare Flüssigkeit ein Wachs oder ein Paraffin ist.

35. Verwendung eines hoch dispersen Triterpens mit einer mittleren Partikelgröße von weniger als 50 µm als Verdickungsmittel in einer unpolaren Flüssigkeit, indem das Triterpen in der Flüssigkeit in einer Konzentration unterhalb einer für die Flüssigkeit und das Triterpen gegebenen Geliergrenze verwendet wird.

36. Verwendung nach Anspruch 35, bei dem die mittlere Partikelgröße des wenigstens einen Triterpens weniger als 10µm beträgt.

37. Verwendung nach einem der Ansprüche 35 oder 36, bei dem ein Anteil an Sekundäragglomeraten des wenigstens einen Triterpens weniger als 20 Gew.% beträgt.

38. Verwendung nach Anspruch 37, bei dem das wenigstens eine Triterpen eine homogene Partikelgrößenverteilung besitzt.

39. Verwendung nach einem der Ansprüche 35 bis 38, bei dem die spezifische Oberfläche des wenigstens einen Triterpens zwischen 1 m²/g und 500 m²/g beträgt.

40. Verwendung nach Anspruch 39, bei dem die spezifische Oberfläche des wenigstens einen Triterpens zwischen 10 m²/g und 100 m²/g beträgt.

41. Verwendung nach Anspruch 40, bei dem die spezifische Oberfläche des wenigstens einen Triterpens zwischen 20 m²/g und 50 m²/g beträgt.

42. Verwendung nach einem der Ansprüche 35 bis 41, bei dem das wenigstens eine Triterpen einen Anteil von mehr als 80 Gew.% Betulin umfasst.

## Claims

1. Use of at least one highly dispersed triterpene having an average particle size of less than 50 µm as an oleogel-forming agent in an oleogel.

2. The use as recited in Claim 1,
wherein the average particle size of the at least one triterpene is less than 10 µm.

3. Use as recited in one of the preceding claims, wherein the proportion of secondary agglomerates of the at least one triterpene is less than 20% by weight.

4. Use as recited in claim 3, wherein the at least one triterpene has a homogeneous particle size distribution.

5. Use as recited in one of the preceding claims, wherein the specific surface area of the at least one triterpene is between 1 m²/g and 500 m²/g.

6. Use as recited in Claim 5, wherein the specific surface area of the at least one triterpene is between 10 m²/g and 100 m²/g.

7. Use as recited in Claim 6, wherein the specific surface area of the at least one triterpene is between 20 m²/g and 50 m²/g.

8. The use as recited in one of the preceding claims,
wherein the at least one triterpene has a proportion of more than 80% by weight betulin.

9. An oleogel, comprising:
- a nonpolar liquid having a proportion between 80% by weight and 99% by weight, based on the total weight of the gel,
- at least one highly dispersed triterpene having an average particle size of less than 50 µm as an oleogel-forming agent in a proportion between 1% by weight and 20% by weight, based on the total weight of the gel.

10. The oleogel as recited in Claim 9,
wherein the average particle size of the at least one triterpene is less than 10 µm.

11. The oleogel as recited in one of claim 9 or 10, wherein the proportion of secondary agglomerates of the at least one triterpene is less than 20% by weight.

12. The oleogel as recited in claim 11, wherein the at least one triterpene has a homogeneous particle size distribution.

13. The oleogel as recited in one of claims 9 to 12, wherein the specific surface area of the at least one triterpene is between 1 m²/g and 500 m²/g.

14. The oleogel as recited in Claim 13, wherein the specific surface area of the at least one triterpene is between 10 m²/g and 100 m²/g.

15. The oleogel as recited in Claim 14, wherein the specific surface area of the at least one triterpene is between 20 m²/g and 50 m²/g.

16. The oleogel as recited in one of claims 9 to 15, wherein the at least one triterpene has a proportion of more than 80% by weight betuline.

17. The oleogel as recited in one of claims 9 to 16, wherein the proportion of the oleogel-forming agent is between 3% by weight and 15% by weight.

18. The oleogel as recited in Claim 17, wherein the proportion of the nonpolar liquid is between 88% by weight and 94% by weight and the proportion of the oleogel-forming agent is between 6% by weight and 12% by weight.

19. The oleogel as recited in one of Claims 9 to 18,
wherein the nonpolar liquid is a vegetable, animal, mineral, or synthetic oil.

20. The oleogel as recited in Claim 19, wherein the oil is one of the following vegetable oils or a mixture of the following vegetable oils: sunflower oil, olive oil, avocado oil, almond oil.

21. The oleogel as recited in one of Claims 9 to 18, wherein the nonpolar liquid is a wax or a paraffin.

22. A method for producing an oleogel, comprising mixing only the following ingredients:
- a nonpolar liquid in a proportion between 80% by weight and 99% by weight, based on the total weight of the gel,
- at least one highly dispersed triterpene having an average particle size of less than 50 µm as an oleogel-forming agent in a proportion between 1% by weight and 20% by weight, based on the total weight of the gel.

23. The method as recited in Claim 22,
wherein the average particle size of the at least one triterpene is less than 10 µm.

24. The method as recited in one of claim 22 or 23, wherein the proportion of secondary agglomerates of the at least one triterpene is less than 20% by weight.

25. The method as recited in 24, wherein the at least one triterpene has a homogeneous particle size distribution.

26. The method as recited in one of claims 22 to 25, wherein the specific surface area of the at least one triterpene is between 1 m²/g and 500 m²/g.

27. The method as recited in Claim 26, wherein the specific surface area of the at least one triterpene is between 10 m²/g and 100 m²/g.

28. The method as recited in Claim 27, wherein the specific surface area of the at least one triterpene is between 20 m²/g and 50 m²/g.

29. The method as recited in one of claims 22 to 28, wherein the at least one triterpene has a proportion of more than 80% by weight betuline.

30. The method as recited in one of claims 22 to 29, wherein the proportion of oleogel-forming agent is between 3% by weight and 15% by weight.

31. The method as recited in Claim 30, wherein the proportion of the nonpolar liquid is between 88% by weight and 94% by weight and the proportion of the oleogel-forming agent is between 6% by weight and 12% by weight.

32. The method as recited in one of Claims 22 to 31,
wherein the nonpolar liquid is a vegetable, animal, or synthetic oil.

33. The method as recited in Claim 32, wherein the oil is one of the following vegetable oils or a mixture of the following vegetable oils: sunflower oil, olive oil, avocado oil, almond oil.

34. The method as recited in one of Claims 22 to 29 and 31, wherein the nonpolar liquid is a wax or a paraffin.

35. Use of at least one highly dispersed triterpene having an average particle size of less than 50 µm as a thickener in a liquid by using the triterpene in the liquid in a concentration below the gelation limit, which is defined for the liquid and the triterpene.

36. Use as recited in Claim 35,
wherein the average particle size of the at least one triterpene is less than 10 µm.

37. Use as recited in one of claims 35 or 36, wherein the proportion of secondary agglomerates of the at least one triterpene is less than 20% by weight.

38. Use as recited in claim 37, wherein the at least one triterpene has a homogeneous particle size distribution.

39. Use as recited in one of claims 35 to 38, wherein the specific surface area of the at least one triterpene is between 1 m²/g and 500 m²/g.

40. Use as recited in Claim 39, wherein the specific surface area of the at least one triterpene is between 10 m²/g and 100 m²/g.

41. Use as recited in Claim 40, wherein the specific surface area of the at least one triterpene is between 20 m²/g and 50 m²/g.

42. Use as recited in one of Claims 35 to 41, wherein the at least one triterpene has a proportion of more than 80% by weight betuline.

## Revendications

1. Utilisation d'au moins un triterpène très dispersé ayant une dimension moyenne de particule plus petite que 50 µm comme agent oléogélifiant dans un oléogel.

2. Utilisation suivant la revendication 1, dans laquelle la dimension moyenne de particule du au moins un triterpène est plus petite que 10 µm.

3. Utilisation suivant l'une des revendications précédentes, dans laquelle une proportion d'agglomérats secondaires du au moins un triterpène est plus petite que 20 % en poids.

4. Utilisation suivant la revendication 3, dans laquelle le au moins un triterpène a une répartition homogène de la dimension des particules.

5. Utilisation suivant l'une des revendications précédentes, dans laquelle la surface spécifique du au moins un triterpène est comprise entre 1 m²/g et 500 m²/g.

6. Utilisation suivant la revendication 5, dans laquelle la surface spécifique du au moins un triterpène est comprise entre 10 m²/g et 100 m²/g.

7. Utilisation suivant la revendication 6, dans laquelle la surface spécifique du au moins un triterpène est comprise entre 20 m²/g et 50 m²/g.

8. Utilisation suivant l'une des revendications précédentes, dans laquelle le au moins un triterpène a une proportion de plus de 80 % en poids de bétuline.

9. Oléogel qui comporte des constituants suivants :
- un liquide non polaire en une proportion comprise entre 80 % en poids et 99 % en poids rapportée au poids total du gel ;
- au moins un triterpène très dispersé comme agent oléogélifiant, qui a une dimension moyenne de particule plus petite que 50 µm en une proportion comprise entre 1 % en poids et 20 % en poids rapportée au poids total du gel.

10. Oléogel suivant la revendication 9, dans lequel la dimension moyenne des particules du au moins un triterpène est plus petite que 10 µm.

11. Oléogel suivant l'une des revendications 9 ou 10, dans lequel la proportion d'agglomérats secondaires du au moins un triterpène est plus petite que 20 % en poids.

12. Oléogel suivant la revendication 11, dans lequel le au moins un triterpène a une répartition homogène de la dimension des particules.

13. Oléogel suivant l'une des revendications 9 à 12, dans lequel la surface spécifique du au moins un triterpène est comprise entre 1 m²/g et 500 m²/g.

14. Oléogel suivant la revendication 13, dans lequel la surface spécifique du au moins un triterpène est comprise entre 10 m²/g et 100 m²/g.

15. Oléogel suivant la revendication 14, dans lequel la surface spécifique du au moins un triterpène est comprise entre 20 m²/g et 50 m²/g.

16. Oléogel suivant l'une des revendications 9 à 15, dans lequel le au moins un triterpène a une proportion de plus de 80 % en poids de bétuline.

17. Oléogel suivant l'une des revendications 9 à 16, dans lequel la proportion d'agent oléogélifiant est comprise entre 3 % en poids et 15 % en poids.

18. Oléogel suivant la revendication 17, dans lequel la proportion du liquide non polaire est comprise entre 88 % en poids et 94 % en poids et la proportion de l'agent oléogélifiant est comprise entre 6 % en poids et 12 % en poids.

19. Oléogel suivant l'une des revendications 9 à 18, dans lequel le liquide non polaire est une huile végétale, animale, minérale ou de synthèse.

20. Oléogel suivant la revendication 19, dans lequel l'huile est l'une des suivantes ou un mélange des huiles végétales suivantes : l'huile de tournesol, l'huile d'olive, l'huile d'avocat, l'huile d'amande.

21. Oléogel suivant l'une des revendications 9 à 18, dans lequel le liquide non polaire est une cire ou une paraffine.

22. Procédé de production d'un oléogel, qui comprend le mélange seulement des constituants suivantes :
- un liquide non polaire en une proportion comprise entre 80 % en poids et 99 % en poids rapportée au poids total du gel ;
- au moins un triterpène très dispersé comme agent oléogélifiant, qui a une dimension moyenne de particule plus petite que 50 µm en une proportion comprise entre 1 % en poids et 20 % en poids rapportée au poids total du gel.

23. Procédé suivant la revendication 22, dans lequel la dimension moyenne des particules de au moins un triterpène est plus petite que 10 µm.

24. Procédé suivant l'une des revendications 22 ou 23, dans lequel une proportion des agglomérats secondaires du au moins un triterpène est plus petite que 20 % en poids.

25. Procédé suivant la revendication 24, dans lequel au moins un triterpène a une répartition homogène de la dimension des particules.

26. Procédé suivant l'une des revendications 22 à 25, dans lequel la surface spécifique du au moins un triterpène est comprise entre 1 m²/g et 500 m²/g.

27. Procédé suivant la revendication 26, dans lequel la surface spécifique du au moins un triterpène est comprise entre 10 m²/g et 100 m²/g.

28. Procédé suivant la revendication 27, dans lequel la surface spécifique du au moins un triterpène est comprise entre 20 m²/g et 50 m²/g.

29. Procédé suivant l'une des revendications 22 à 28, dans lequel le au moins un triterpène a une proportion de plus de 80 % en poids de bétuline.

30. Procédé suivant l'une des revendications 22 à 29, dans lequel la proportion d'agent oléogélifiant est comprise entre 3 % en poids et 15 % en poids.

31. Procédé suivant la revendication 30, dans lequel la proportion du liquide non polaire est comprise entre 88 % en poids et 94 % en poids et la proportion de l'agent oléogélifiant est comprise entre 6 % en poids et 12 % en poids.

32. Procédé suivant l'une des revendications 22 à 31, dans lequel le liquide non polaire est une huile végétale, animale, minérale ou de synthèse.

33. Procédé suivant la revendication 32, dans lequel l'huile est l'une des suivantes ou un mélange des huiles végétales suivantes : l'huile de tournesol, l'huile d'olive, l'huile d'avocat, l'huile d'amande.

34. Procédé suivant l'une des revendications 22 à 29 et 31, dans lequel le liquide non polaire est une cire ou une paraffine.

35. Utilisation d'un triterpène très dispersé ayant une dimension moyenne des particules plus petite que 50 µm comme agent épaississant d'un liquide non polaire en utilisant le triterpène dans le liquide en une concentration inférieure à une limite de gélification donnée pour le liquide et le triterpène.

36. Utilisation suivant la revendication 35, dans laquelle la dimension moyenne des particules de au moins un triterpène est plus petite que 10 µm.

37. Utilisation suivant l'une des revendications 35 ou 36, dans laquelle une proportion des agglomérats secondaires du au moins un triterpène est plus petite que 20 % en poids.

38. Utilisation suivant la revendication 37, dans laquelle le au moins un triterpène a une répartition homogène de la dimension des particules.

39. Utilisation suivant l'une des revendications 35 à 38, dans laquelle la surface spécifique du au moins un triterpène est comprise entre 1 m²/g et 500 m²/g.

40. Utilisation suivant la revendication 39, dans laquelle la surface spécifique du au moins un triterpène est comprise entre 10 m²/g et 100 m²/g.

41. Utilisation suivant la revendication 40, dans laquelle la surface spécifique du au moins un triterpène est comprise entre 20 m²/g et 50 m²/g.

42. Utilisation suivant l'une des revendications 35 à 41, dans laquelle le au moins un triterpène a une proportion de plus de 80 % en poids de bétuline.
